# EUROPÄISCHE PATENTSCHRIFT

(11) **EP 1 112 456 B1**
(45) Veröffentlichungstag und Bekanntmachung des Hinweises auf die Patenterteilung: **26.02.2003**
(21) Anmeldenummer: 99947311.9
(22) Anmeldetag: 10.09.1999
(51) Int. Cl.: F16L 1/00

(54) **SCHLAUCH ZUR AUSKLEIDUNG VON ROHRLEITUNGEN UND KANÄLEN SOWIE VERFAHREN UND VORRICHTUNG ZU SEINER HERSTELLUNG**
TUBE FOR LINING PIPES AND CHANNELS, AND A METHOD AND DEVICE FOR THE PRODUCTION THEREOF
FLEXIBLE POUR GARNIR DES CANALISATIONS ET DES CANAUX, ET PROCEDE ET DISPOSITIF PERMETTANT DE LE PRODUIRE

(30) Priorität: 11.09.1998 US 151511
(43) Veröffentlichungstag der Anmeldung: 04.07.2001
(73) Patentinhaber: Karl Weiss Hoch-, Tief- und Rohrleitungsbau GmbH & Co., 14163 Berlin (DE)
(72) Erfinder: SCHWERT, Siegfried, D-14195 Berlin (DE); RABOLD, Wolf, D-13187 Berlin (DE); MARQUARDT, Peter, D-13627 Berlin (DE)
(74) Vertreter: Pfenning, Meinig & Partner
(86) Internationale Anmeldenummer: EP9906702
(87) Internationale Veröffentlichungsnummer: WO00015992

(56) Entgegenhaltungen:
- EP-A- 0 294 008
- DE-A- 2 121 145
- DE-U- 9 105 040
- US-A- 3 436 447
- US-A- 4 576 205
- US-A- 4 740 261
- US-A- 4 871 413
- US-A- 5 164 237

## Beschreibung

Die vorliegende Erfindung betrifft einen Schlauch zur Auskleidung von Rohrleitungen und Kanälen sowie ein Verfahren und eine Vorrichtung zu seiner Herstellung.

Es ist bekannt, außen beschichtete Schläuche zur Auskleidung einer Rohrleitung mit einer Öffnung am Rohranfang umstülpend in diese Rohrleitung einzubringen, wobei die Außenfläche des Schlauches umgestülpt wird und die Innenwand der sanierten Rohrleitung bildet. Der Schlauch kann jedoch auch durch Einziehen (ohne Umstülpung) in eine Rohrleitung oder einen Kanal eingebracht werden.

Mit Schläuchen nach dem Stand der Technik ist es jedoch, besonders bei kleineren Rohrdurchmessern, nicht möglich, auch kleinste Bögen und Winkelstücke faltenfrei auszukleiden. Bei Schläuchen nach dem Stand der Technik bilden sich in diesen Fällen Stauchfalten im Bereich von Krümmern, wodurch die Bedingungen für durchzuleitende Medien stark verschlechtert werden. So werden in Druckleitungen an diesen Stellen hohe Druckverluste verursacht und bei Abwasserleitungen können Ablagerungen an den Stauchfalten zu Verstopfungen führen. Aufgrund dieser Stauchfaltenbildung war der Einsatz der oben beschriebenen, im Umstülpverfahren einzubringenden, Schläuche bisher nur möglich, wenn es sich um geradlinige Leitungsabschnitte oder Bögen mit großem Rohrdurchmesser bzw. mit großen Radien handelte.

Aus der US-A-4576205 ist ein Schlauch zur Auskleidung von Rohrleitungen und Kanälen bekannt, welcher aus einem beschichteten Gewebe aus Kettfäden relativ hoher Elastizität und Schußfäden relativ geringer Elastizität besteht. Die Längsdehnbarkeit sollte zwischen 10 und 100% liegen und die Dehnbarkeit in radialer Richtung sollte so groß sein, daß ein Schlauch, dessen Durchmesser im unbelasteten Zustand 80-100% des Innendurchmessers der Rohrleitung beträgt, bei dem Druck, der zum Umstülpen des Schlauches beim Einbringen in die Rohrleitung verwendet wird, auf etwa 100-130% des Innendurchmessers der Rohrleitung gedehnt wird.

Weiterhin wird in der US-A-5164237 ein Schlauch zur Auskleidung von Rohrleitungen offenbart, dessen Kettfäden vorzugsweise aus Polyester mit einer maximalen Längsdehnbarkeit von etwa 10% bestehen. Die Schußfäden hingegen sollen eine hohe Festigkeit mit einer Dehnbarkeit in Längsrichtung etwa zwischen 0,6 und 4,2% haben. Hierdurch wird ein Schlauch erhalten, der eine hohe Widerstandskraft bei Erdversetzungen oder Erdbeben besitzt.

Schließlich wird in der US-A-3436 447 ein Verfahren zur Herstellung eines Schlauches aus einem beschichteten Gewebe beschrieben, bei dem eine Kunststoffschmelze in eine Kammer gepreßt und das schlauchförmige Gewebe in flachem Zustand in seiner Längsrichtung und in seinem Inneren ungestützt durch die Kammer geführt werden. Hierbei wird das Gewebe auf seinen beiden Außenseiten mit der Kunststoffschmelze beschichtet. Die Druckschrift enthält keine Angaben über die Dehnbarkeit des Schlauches.

Ausgehend von diesem Stand der Technik ist es die Aufgabe der vorliegenden Erfindung, einen Schlauch zur Auskleidung von Rohrleitungen und Kanälen, welcher in eine Rohrleitung eingebracht wird, wobei der Schlauch aus einem beschichteten Gewebe aus hochelastischen Kettfäden sowie Schußfäden geringerer Elastizität besteht, sowie ein Verfahren und eine Vorrichtung zu seiner Herstellung zu schaffen, welcher auch bei geringen Rohrdurchmessern, wie sie etwa bei Hausanschlußleitungen für Gas bzw. Trinkwasser oder Abwasser vorkommen, faltenfrei auch in engen Biegungen verlegbar ist.

Diese Aufgabe wird in bezug auf den Schlauch durch Patentanspruch 1, bezüglich des Herstellverfahrens durch Patentanspruch 20 sowie bezüglich der Vorrichtung zur Herstellung des Schlauches durch Patentanspruch 28 gelöst.

Dadurch, daß das Verhältnis von E max der Kettfäden zu E max der Schußfäden im Bereich von 12 bis 40 liegt, ist es durch Anordnung der Schlauchlängsachse in Richtung der Längsachse der Kettfäden möglich, einen Schlauch mit hoher Längsdehnung zur Verfügung zu stellen. Hiermit können auch engste Krümmungen von Rohrleitungen nahezu faltenfrei ausgekleidet werden. Dies liegt daran, daß durch die verbesserte Längsdehnbarkeit des Schlauches auch im Bereich der vom Krümmungsmittelpunkt der Rohrbiegung entferntere seitliche Schlauchabschnitt stärker gedehnt werden kann, so daß es zu einem flachen Auskleiden der Rohrinnenwand auch im Bereich dieser Außenkrümmung kommen kann. Außerdem erlaubt die hohe Elastizität der Kettfäden bei dem mit Druck durchgeführten Umstülpverfahren niedrigere "Reversionsdrücke" zum Umstülpen des Schlauches.

Trotz des vorgenannten Verhältnisses ergibt sich noch eine ausreichende Elastizität der Schußfäden, welche für die Querdehnbarkeit des beschichteten Schlauches mitverantwortlich sind. Diese Querdehnbarkeit, welche erforderlich ist, um mit bereits niedrigen Reversionsdrücken ein komplettes Anlegen des Schlauches an die Rohrwand zu erreichen, ist bei einem erfindungsgemäßen Schlauch daher stets erfüllt. Die Längsdehnung des erfindungsgemäßen Schlauches ist besonders hoch, wenn diese mit dem erfindungsgemäßen Herstellungsverfahren bzw. der erfindungsgemäßen Herstellungsvorrichtung hergestellt wird.

Dadurch, daß zur Herstellung des Schlauches Kunststoffschmelze in eine Kammer gepreßt und ein Schlauchrohling entlang seiner Längsachse und im Inneren ungestützt durch die Kammer geführt wird, wobei die Außenfläche des Schlauchrohlings mit Kunststoffschmelze beschichtet wird, bleibt die hohe Elastizität der vorteilhafterweise in Längsrichtung angeordneten hochelastischen Kettfäden auch nach dem Beschichtungsvorgang in hohem Maße erhalten. Trotzdem ist die Güte der Beschichtung sehr hoch. Durch das druckbehaftete Aufprägen der Kunststoffschmelze kommt es nicht, wie etwa bei Tauchverfahren, zur Blasenbildung in der Beschichtung, welche besonders nach dem Umstülpvorgang eine Beeinträchtigung der Schlauchdichte bewirken kann.

Im Gegensatz zur Flachbeschichtung, bei welcher ein flachgefalteter Schlauch auf der Oberseite und der Unterseite mit einer Beschichtung aufgetragen wird, ist die Oberflächengüte bei dem erfindungsgemäßen Herstellungsverfahren deutlich verbessert. Der Grund dafür ist wie folgt: Bei der Nachbeschichtung der Seitenkanten bei herkömmlicher Flachbeschichtung ist es einerseits möglich, deutlich über die Schlauchbreite hinweg die Schlauchbeschichtung vorzunehmen und den seitlich überstehenden Teil in einem weiteren Arbeitsgang abzutrennen (dies ist jedoch aufwendig und qualitativ unzureichend). Die andere Möglichkeit, bei herkömmlicher Flachbeschichtung im Bereich der Seitenkanten die fehlende Beschichtung aufzubringen, ist ein Umlegen bzw. Drehen des Schlauches und eine spätere Nachbeschichtung der dann flachliegenden ehemaligen Seitenkante. Hierbei kommt es jedoch zur Ausbildung einer Doppelung und somit einer ungleichmäßigen Beschichtungsstärke, sodaß ein späteres Anliegen des Schlauches in der Rohrinnenwand selbst bei Aufblähen des Schlauches unter hoher Druckbelastung nicht möglich ist (es besteht sogar die Gefahr von Hinterwanderungen bzw. einer Fehlsanierung). Da die Querelastizität im Bereich der Doppelung geringer ausfällt, ist die Gefahr, daß der Schlauch in diesem Bereich nicht anliegt, in hohem Maße gegeben.

Bei Beschichtungsverfahren nach dem Stand der Technik mittels Extrusionswerkzeugen wird hingegen ein Schlauchrohling zur Erhaltung seines Kreisquerschnittes über einen entsprechenden Dorn gezogen, während die Kunststoffschmelze auf die Außenfläche des Schlauchrohlings aufgebracht wird. Durch den Außendruck der Kunststoffschmelze wird der Schlauchrohling hierbei jedoch radial mit hohem Druck gegen den Dorn gepreßt, so daß hohe Zugkräfte in Längsrichtung des Schlauchrohlings nötig sind, um den Schlauchrohling durch das Extrusionswerkzeug zu ziehen. Bedingt durch diese starke Zugbelastung des Schlauchrohlings kommt es zu einer starken Längsdehnung des Schlauchrohlings während des Beschichtungsvorganges. Dies führt dazu, daß bei dem Erkalten der als Beschichtung aufgebrachten Kunststoffschmelze die Längsdehnung des Schlauchrohlings quasi "eingefroren" wird, so daß im erkalteten Zustand der beschichtete Schlauch lediglich eine geringe Restdehnbarkeit in Längsrichtung aufweist.

Vorteilhafte Ausführungsformen der vorliegenden Erfindung werden in den übrigen Patentansprüchen beschrieben.

Eine weitere Variante der Erfindung sieht vor, daß der zu beschichtende Schlauch kein gewebter, sondern aus einem hochelastischen Garn gewirkter (d.h. in einem Strickverfahren hergestellter) Schlauch ist. Hiermit ist es möglich, mittels einer einzigen Garnart einen Gewebeschlauch mit hoher Längs- und Querdehnung herzustellen.

Eine besonders vorteilhafte Ausführungsform der Erfindung sieht vor, daß ein Kettfaden aus einem mit einem Umwindungsgarn umwundenen Grundkettfaden besteht, wobei der Grundkettfaden eine längenbezogene Masse im Bereich von 22 bis 4000 dtex und das Umwindungsgarn eine längenbezogene Massen von 44 bis 1000 dtex (1 dtex = 0,1 Milligramm pro Meter) aufweist. Bevorzugte Materialien sind für den Grundkettfaden Polyurethan und für das Windungsgarn Polyester oder Polyamid. Hiermit wird ein ungelöstes Problem beim Auskleiden von Rohrleitungen, welche gerade und gekrümmte Abschnitte aufweisen, gelöst. Dieses Problem bestand darin, daß bei geraden Rohrabschnitten (um einen kontrollierten Vortrieb zu ermöglichen) eine große Längsdehnung nicht erwünscht, jedoch im Bereich von Krümmungen wünschenswert war. Der erfindungsgemäße Schlauch mit einem Grundkettfaden sowie einem Umwindungsgarn weist, um ein kontrolliertes Einbringen des Schlauches in geraden Rohrabschnitten zu gewährleisten, hierzu eine begrenzte Längsdehnung auf, da das Umwindungsgarn den Grundkettfaden stabilisiert. Aufgrund des sehr elastischen Grundkettfadens ist jedoch in Krümmungen, in denen eine Ausdehnung des Schlauches für das flächige Anliegen des Schlauches an der Rohrinnenwand nötig ist, eine ausreichende Dehnbarkeit des Schlauches gegeben, die durch eine Krafterhöhung beim Durchfahren der Bögen aktiviert wird.

Eine weitere vorteilhafte Weiterbildung der Erfindung sieht vor, daß die Beschichtung des Schlauches radial nur bis zu einem Teil, z.B. der Hälfte, der Dicke des Gewebes eingebracht ist. Hiermit wird einerseits eine ausreichende Verankerung der Beschichtung im Gewebe erreicht und andererseits die Durchtränkung der Gewebeinnenfläche, durch Klebstoffe zum Anbringen des Schlauches an der Rohrwand, ermöglicht.

Eine weitere vorteilhafte Ausführungsform sieht vor, daß die Kammer zur Aufbringung der Kunststoffschmelze auf einen Schlauchrohling quer zur Längsrichtung des durchgeführten Schlauchrohlings einen zu dem Querschnitt des infolge des Druckes der Kunststoffschmelze flachliegenden Schlauchrohlings korrespondierenden Querschnitt aufweist. Hierdurch wird die Kammer der Querschnittsgeometrie des Schlauches angepaßt, so daß die Wege der Kunststoffschmelze von den Wänden der Kammer zu der Schlauchaußenfläche hin klein gehalten werden und Toträume der Kunststoffschmelze vermieden werden können.

Eine besonders vorteilhafte Ausführungsform sieht vor, daß die in das Kammerinnere führenden Schmelzeleitungen während ihres Verlaufes in der Kammer, also in ihrem zu dem Schlauchrohling hin orientierten Endbereich, als Wendelverteiler ausgeführt sind. Hierbei wird die Kunststoffschmelze aus mehreren, über den Umfang des Schlauchrohlingquerschnittes verteilten Stellen zugeführt, so daß eine über den Umfang des Schlauchrohlings gleichförmige Beschichtung gewährleistet werden kann.

Weitere vorteilhafte Ausführungsformen der vorliegenden Erfindung werden in den verbleibenden Patentansprüchen gegeben.

Die Erfindung wird nun anhand mehrerer Figuren erläutert. Es zeigen:
- Fig. 1a: einen Auskleidungsvorgang bei gekrümmten Rohren mit einem Schlauch nach dem Stand der Technik,
- Fig. 1b: einen Auskleidungsvorgang bei gekrümmten Rohren mit einem erfindungsgemäßen Schlauch,
- Fig. 2a: Draufsicht eines Schlauchgewebes,
- Fig. 2b: einen Schnitt B-B des Schlauchgewebes aus Fig. 2a,
- Fig. 2c: einen Schnitt C-C des Schlauchgewebes aus Fig. 2a,
- Fig. 2d: einen Schnitt C-C des Schlauchgewebes aus Fig. 2a im gedehnten Zustand,
- Fig.3: der Herstellungsablauf eines erfindungsgemäßen Schlauches,
- Fig. 4a: ein Beschichtungswerkzeug nach dem Stand der Technik, und
- Fig. 4b: Ansichten eines erfindungsgemäßen Beschichtungswerkzeuges.

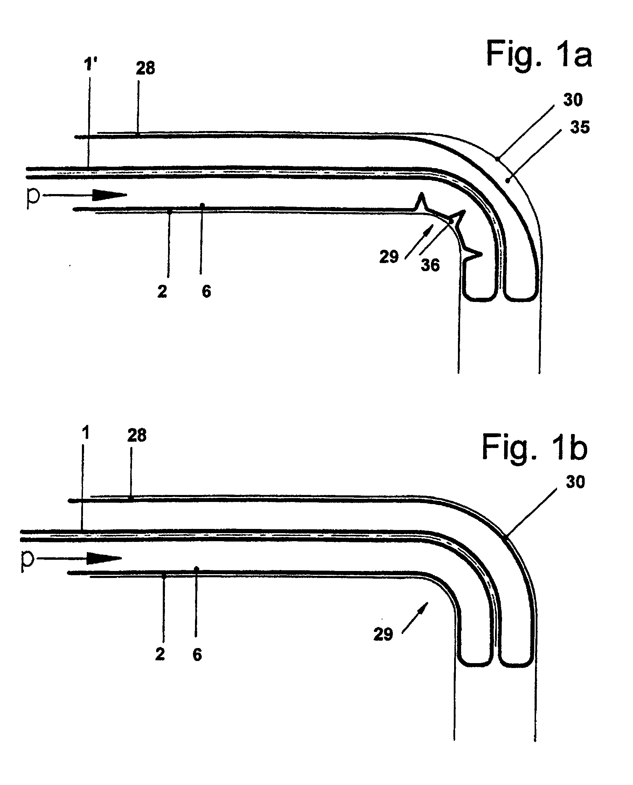

Fig. 1a zeigt eine Rohrleitung 2, in welche ein Schlauch 1' unter Einleitung eines Druckes p umstülpend eingebracht wird. Die noch nicht umgestülpten Abschnitte des Schlauches 1', z.B. die noch nicht in der Rohrleitung 2 befindlichen, sind auf der Außenseite mit einer Beschichtung (im allgemeinen aus Kunststoff) versehen. Die Innenfläche des noch nicht gestülpten Schlauches 1' ist mit einem Klebstoff 28 versehen, welcher nach dem Umkrempeln des Schlauches an der Innenwandung des Rohres 2 haftet und somit einen dauerhaften Halt des Schlauches zu der Rohrleitung 2 bildet. Im Bereich von Krümmungen 29 der Rohrleitung 2 kommt es jedoch zu Fehlsanierungen. Im Bereich des inneren Krümmungsradius kommt es zur Faltenbildung 36 des Schlauches nach dem Stand der Technik. Darüber hinaus kann ein nicht vollständiges Anliegen des Schlauches im Bereich des äußeren Krümmungsradius auftreten (nicht ausgekleideter Bereich 35).

Fig. 1b zeigt die unter einem Druck p in das Innere der Rohrleitung 2 erfolgende umstülpende Vorantreibung eines erfindungsgemäßen Schlauches 1. Hierbei ist der ungestülpte Schlauch 1 an seiner Innenfläche mit einem Klebstoff 28 zur späteren Anhaftung an der Rohrinnenwand bestrichen, die ungestülpte Außenfläche ist mit einer Beschichtung aus einem thermoplastischen Kunststoff versehen, welche druck- und wasserdicht ist sowie gegen Chemikalien und künstliche Alterung beständig ist. Als Material für die Beschichtung kommt vorzugsweise ein PolyurethanElastomer in Betracht. Der Schlauch zeichnet sich insbesondere auch durch eine hohe Festigkeit gegen mechanische Belastungen jeglicher Art aus und weist trotz seiner hohen Flexibilität den erforderlichen Berstdruck auf, der somit ein sicheres Umstülpen auch mit einem ausreichend hohen Druck p ermöglicht. Das Verhältnis zwischen dem Berstdruck des Schlauches 1 (unabhängig von seiner Stülpung) und dem Reversionsdruck p zum druckbehafteten Umstülpen des Schlauches 1 in die Rohrleitung 2 liegt vorzugsweise im Bereich zwischen 1,1 und 4,4, besonders vorzugsweise zwischen 1,5 und 3,5.

Die Beschichtung 6 des Schlauches ist radial nur bis zu einem Teil der Dicke des einen Schlauchrohling 10 bildenden Gewebes bzw. Gewirkes eingebracht. Damit ist es ermöglicht, daß ein Klebstoff 28 auf der der Beschichtung 6 entgegengesetzten Fläche des Gewebes bzw. Gewirkes ausreichend tief in das Gewebe bzw. Gewirke eindringen kann. Außerdem besitzt das Gewebe bzw. Gewirke eine gute Aufnahme- bzw. Durchdringungsfähigkeit für den Klebstoff, sodaß eine spätere Hinterwanderung mit Gasen (d.h. ein Eindringen von Gasen in den Raum zwischen Schlauch und Rohrleitung) sowie ein Wandern des Gases im Gewebe ausgeschlossen werden kann.

Der Schlauch 1 legt sich auch in Krümmungen 29 der Rohrleitung 2 über den gesamten Umfang der Rohrleitung hinweg vollflächig an die Innenwand der Rohrleitung an. Dies erfordert insbesondere bei recht- oder spitzwinkligen Rohrbiegungen eine sehr starke Längsdehnung des Schlauchmaterials im Bereich des äußeren Krümmungsradius 30 der Rohrinnenwand. Der Schlauch 1 weist ein εₘₐₓ in Längsrichtung im Bereich von 40 bis 400 % auf. (εₘₐₓ ist, wie üblich, in der vorliegenden Anmeldung definiert als das Verhältnis der maximalen Schlauchlängenzunahme zu der Gesamtlänge des Schlauches bei einem Zugversuch im Zeitpunkt des Bruches, hierbei greift eine Zugkraft Fₘₐₓ am Prüfkörper an). εₘₐₓ liegt für den Schlauch 1 bevorzugt im Bereich von 80 bis 300 % (zum Vergleich: εₘₐₓ eines Schlauches nach dem Stand der Technik 1' liegt bei maximal 35 bis 40 %).

Neben der hervorragenden Längsdehnung weist der erfindungsgemäße Schlauch 1 auch eine ausreichende Querdehnung auf, um Schwankungen des Rohrinnendurchmessers besser ausgleichen zu können. Bei radialer Zugbelastung des Schlauches 1 bewegt sich εₘₐₓ im Bereich zwischen 50 und 100 %, vorzugsweise im Bereich zwischen 60 und 90 % (je nach Beschichtungsstärke). Hierbei beträgt Fₘₐₓ vorzugsweise zwischen 50 und 70 N pro mm Schlauchbreite. Das Verhältnis von ε_{max,radial} zu ε_{max,axial} liegt vorzugsweise zwischen 1,1 und 10.

Bei einer Temperatur von 80° C liegt der Berstdruck immer noch zwischen 60 und 80 % des Berstdrucks bei Raumtemperatur. Hieraus ergibt sich, daß selbst bei hohen Temperaturen bei der Rohrauskleidung der Reversionsdruck p stets deutlich kleiner ist als der Berstdruck des Schlauches, so daß es keinesfalls zu von Druck verursachten Beschädigungen des Schlauches kommen kann.

Selbstverständlich ist das Umstülpverfahren nicht zwingend, der erfindungsgemäße Schlauch kann auch nach anderen Methoden, etwa durch Einziehen, in Rohrleitungen oder Kanäle (auch mit nichtkreisförmigem, z.B. ellipsoidem) Querschnitt eingezogen werden. Der Außendurchmesser des Schlauches 1 im ungedehnten Zustand kann zwischen 9 und 1200 mm, vorzugsweise zwischen 12 und 200 mm liegen.

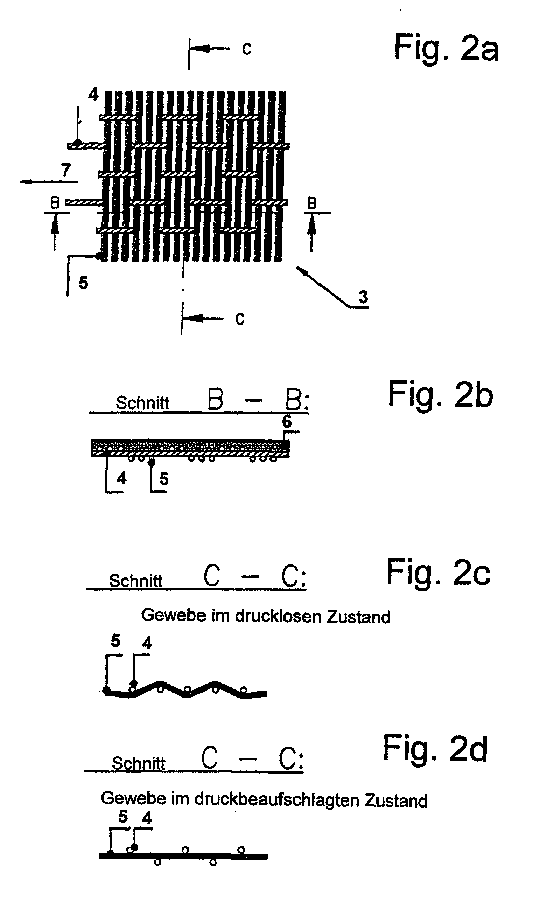

Im folgenden wird der Aufbau des Schlauches 1 anhand der Figuren 2a bis 2c näher erläutert. FIG. 2a zeigt ein Gewebe aus Kettfäden 4 und Schußfäden 5. In FIG. 2a ist der Einfachheit halber das Gewebe in einem ebenen Zustand dargestellt. Ein Schlauchrohling 10, welcher die Struktur des Gewebes 3 aufweist, wird aus Kettfäden 4 und Schußfäden 5 auf einem speziellen Rundwebstuhl gewebt, wobei die Kettfäden 4 nebeneinanderliegend und längsgerichtet in Form eines Rohres angeordnet werden und ein bzw. mehrere Schußfäden bezüglich der Rohrform spiralförmig und in vorbestimmter Abfolge unter den Kettfäden 4 hindurch und über diese hinweg, unter Bildung eines Gewebeschlauches,geführt werden.

Selbstverständlich ist es auch möglich, den Schlauchrohling 10 als ein Gewirke herzustellen, d.h. aus einem Garn zu stricken. Eine weitere Möglichkeit besteht darin, den Schlauchrohling 10 aus einem im wesentlichen rechteckförmigen ebenen Gewebe oder einem anderen textilen Gebilde, etwa Vlies oder Filz, durch Zusammenfügen der Längsseiten des Rechtecks zu einer Rohrform zu bilden.

Das in Fig. 2a gezeigte Gewebe 3 ist als "Leinwandbindung" ausgeführt. Hierbei wird ein Schußfaden 5 wechselweise unterhalb und oberhalb von längsgerichtet benachbarten Kettfäden geführt. Selbstverständlich ist es auch möglich, andere Webarten (Körperbindung, Atlasbindungen) vorzusehen, beispielsweise den Schußfaden unter mehreren benachbarten Kettfäden hindurchzuführen und nachfolgend über mehrere Kettfäden hinwegzuführen. In dem Gewebe 3 sind im flachen Zustand des Gewebes 10 bis 1000 Kettfäden pro Dezimeter, vorzugsweise 50 bis 300 pro Dezimeter, längsgerichtet benachbart. Es sind 10 bis 1000 Schußfäden pro Dezimeter, vorzugsweise 50 bis 300 pro Dezimeter entspannten Gewebes, längsgerichtet benachbart. Bei dem in Fig. 2a gezeigten Gewebe sind drei Schuß jeweils parallel gemeinsam als Schuß geführt, vorzugsweise können auch vier Schußfäden oder eine beliebige andere Anzahl von Schußfäden parallel gemeinsam als Schuß geführt werden. εₘₐₓ des Gewebes 3 bei Zug in Richtung der Kettfäden 4 liegt im Bereich von 5 bis 500 %, vorzugsweise im Bereich von 100 bis 400 %. Besonders kennzeichnend für den erfindungsgemäßen Schlauch ist, daß die Kettfäden hochelastisch sind, während die Schußfäden eine geringere Elastizität aufweisen. Hierbei liegt das Verhältnis von εₘₐₓ der Kettfäden zu εₘₐₓ der Schußfäden vorzugweise im Bereich von 1,1 bis 50, besonders vorzugsweise im Bereich von 12 bis 40.

Alternativ ist es auch möglich, den erfindungsgemäßen Schlauch auf einem beschichteten Gewirke aus hochelastischem Garn herzustellen, wobei das Garn in etwa die Eigenschaften der Kettfäden 4 besitzt.

Die Kettfäden 4 können aus vulkanisierten Elastomeren oder thermoplastischen Elastomeren bestehen, vorzugsweise sind sie aus Polyurethangarn. Ein einzelner Kettfaden 4 kann auch als Verbund aus verschiedenen Materialien hergestellt sein. Beispielsweise ist es möglich, daß ein Kettfaden 4 aus einem mit einem aus Polyester oder Polyamid bestehenden Windungsgarn umwundenen Grundkettfaden aus Polyurethan besteht (das Umwindungsgarn ist hierbei im Wesentlichen unelastisch, der Grundkettfaden sehr elastisch; wird ein Grundkettfaden mit extrem hoher Elastizität verwendet, ist eine mehrfache Umwindung mit Umwindungsgarn vorzusehen). Hierbei weist der Grundkettfaden vorzugsweise eine längenbezogene Masse im Bereich von 22 bis 4000 dtex und das Umwindungsgarn eine längenbezogene Masse von 44 bis 1000 dtex auf. Es ist beispielsweise möglich, daß ein Grundkettfaden aus einem Polyurethangarn mit 940 dtex besteht, welcher von einem Umwindungsgarn aus Polyamid 66 mit 78 dtex zweifach umwunden ist. Ein auf diese Weise aufgebauter Kettfaden weist ein εₘₐₓ 450 % sowie ein Fₘₐₓ von 9 Newton (gemessen beim Reissen der Umwindungsgarne) auf.

Je nach Ausführungsform kann ein Grundkettfaden einfach oder mehrfach mit einem Umwindungsgarn umwunden sein. Allen Ausführungsformen ist jedoch gemeinsam, daß ein einzelner Kettfaden 4 ein εₘₐₓ im Bereich von 50 bis 500 % und ein Fₘₐₓ von 1 bis 100 Newton aufweist (diese Werte für die Bruchdehnung bzw. die Bruchkraft wurden bei erster Rißbildung der Umwindungsgarne ermittelt).

Die Schußfäden 5 weisen eine längenbezogene Masse von 10 bis 1000 dtex (vorzugsweise 80 bis 550 dtex), ein εₘₐₓ im Bereich von 1 bis 50 % (vorzugsweise 8 bis 30 %) und ein Fₘₐₓ im Bereich von 1 bis 100 Newton (vorzugsweise 5 - 34 Newton) auf. Zur Verbesserung der elastischen Eigenschaften und auch der Saugfähigkeit können Schußfäden und auch Kettfäden gegebenenfalls texturiert sein, es ist auch möglich, die Fäden zu verwirbeln. Als bevorzugte Materialen für die Schußfäden kommt Polyestergarn in Betracht.

Fig. 2b zeigt einen Schnitt B-B durch das Gewebestück aus Fig. 2a. Hierin ist auf der Oberseite des Gewebes 3 außerdem eine Beschichtung 6 aufgebracht. Diese Beschichtung 6 besteht aus einem thermoplastischen Kunststoff hoher Flexibilität. Das εₘₐₓ der Beschichtung beträgt im nicht auf einem Träger beschichteten Zustand zwischen 80 und 800 %, vorzugsweise zwischen 300 und 700 %. Je nach Anwendungszweck kann die Beschichtung, welche die Innenwand eines ausgekleideten Rohres bzw. Kanals bildet, mit unterschiedlichen Werkstoffen beschichtet sein. Im Falle von Gasleitungen und einigen Kanälen kann die Beschichtung z.B. aus einem Polyurethan, insbesondere in seiner Ausführung als thermoplastisches Polyurethan-Elastomer (TPU) oder aus einem Polyester, insbesondere in seiner Ausführung als thermoplastisches Polyether-Ester-Elastomer (TPE) bestehen. Bei der Auskleidung von Trinkwasserrohren und einigen Kanälen ist die Anwendung von Polyolefinen einschließlich Polyethylen (PE) sowie Copolymeren aus Ethylen und anderen α-Olefinen und thermoplastischen Polyolefin-Elastomeren (TPO), und weiterhin von thermoplastischen Styrol-Butadien- bzw. Styrol-Ethylen-Propylen-Copolymeren (TPS) oder Mischungen aus Polypropylen und vernetzten Ethylen-Propylen-Dien-Copolymeren (TPV) vorteilhaft.

Ein besonders häufig angewendetes Beschichtungsmaterial ist thermoplastisches Polyurethan-Elastomer, welches eine hohe Beständigkeit gegenüber Lösungsmitteln aufweist. Dieses hat einen Elastizitätsmodul von 10 MPa, ein **ε**ₘₐₓ von 450 %, eine Bruchspannung von 30 MPa sowie eine Härte von 95 Shore A bzw. 47 Shore D.

Aus Fig. 2c wird besonders deutlich, warum trotz der eher geringen Elastizität der Schußfäden 5 (z.B. bei εₘₐₓ = 12 %) die Querdehnung eines Schlauches aus einem Gewebe 3, wobei die Kettfäden in Schlauchlängsrichtung angeordnet sind, hoch ist. Bei Wirkung eines Überdruckes innerhalb des Schlauches kommt es zunächst zu einer Straffung des Schußfadens 5, wobei über dem Schußfaden geführte Kettfäden 4 hochgepreßt und unterhalb des Schußfadens 5 verlaufende hochelastische Kettfäden 4 hinuntergepreßt werden (siehe auch Fig. 2d). Somit trägt die Elastizität der in Schlauchlängsrichtung 7 angeordneten Kettfäden 4 auch zu einer verbesserten Querdehnung des Schlauches 1 bei. Zusätzlich zu dem oben beschriebenen Umfangszuwachs durch Wegdrücken der Kettfäden durch den Schußfaden 5 kommt es auch zu einer Umfangszunahme des Schlauches 1 durch die Dehnung der Schußfäden 5 selbst.

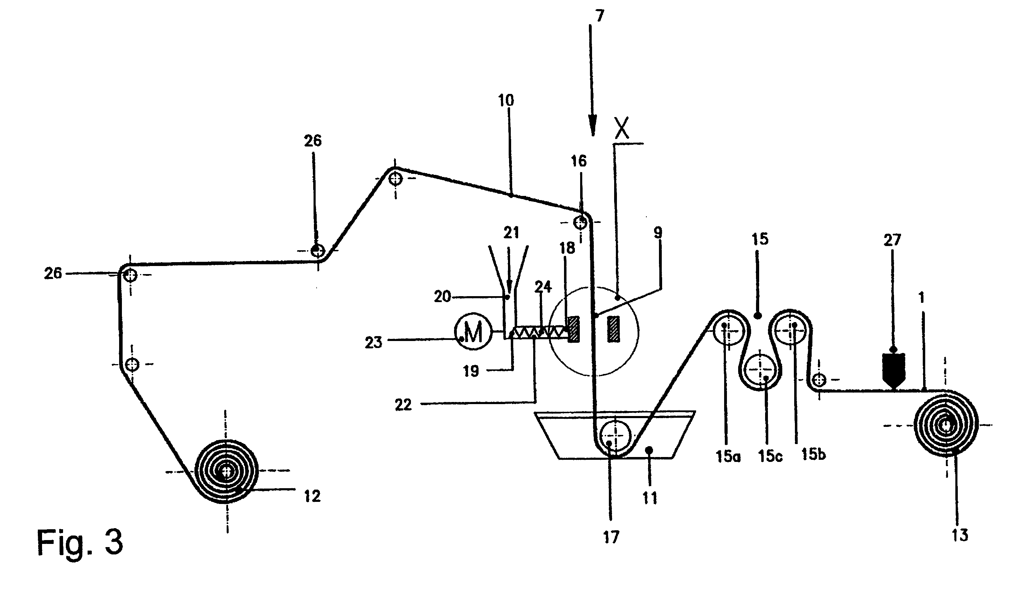

Fig. 3 zeigt den Ablauf eines Verfahrens zur Herstellung eines Schlauches mit hoher Längsdehnung. Auf einer Haspel 12 ist ein Schlauchrohling 10 aufgewikelt. Dieser Schlauchrohling 10 besteht aus einem mit in Längsrichtung des Schlauchrohlings angeordneten Kettfäden 4 bestehenden Gewebe 3 bzw. einem entsprechenden Gewirke. Der Schlauch wird von der Haspel bzw. Schlauchtrommel 12 abgewickelt und über Umlenkwellen 26 in flacher Form geführt. Hiernach wird der Schlauchrohling 10 durch eine Beschichtungskammer 9 gezogen, wobei die Außenoberfläche des Schlauchrohlings mit Kunststoffschmelze beschichtet wird. Der Schlauchrohling 10 ist zwischen den Walzen 16 und 17 gespannt, sodaß er zwischen diesen Walzen einen geraden Verlauf besitzt. Der Schlauchrohling wird allein durch diese Walzen geführt und nicht durch zusätzliche Vorrichtungen im Inneren des Schlauchrohlings gestützt. Am Eingang der Kammer 9 ist ein Schlitz zum Einführen des Schlauchrohlings vorgesehen, welcher auf Form und Größe des flachen Schlauchrohlings 10 so abgestimmt ist, daß nur ein schmaler Spalt zwischen Schlauchrohling-Außenfläche sowie Schlitzinnenseite umlaufend verbleibt. Am Ausgang der Kammer 9 ist ein nicht dargestellter Abstreifer angebracht, welcher mittels zweier Lippen aus Metall, welche auf die Flachseiten des flachen Schlauchrohlings pressen, überschüssige, flüssige Kunststoffschmelze abstreift. Hierdurch wird die Beschichtungsstärke des Schlauches 1 bzw. des beschichteten Schlauchrohlings 10 vergleichmäßigt. Auf die genaue Wirkungsweise der Kammer 9 sowie des angeschlossenen Extruders wird weiter unten genauer eingegangen.

Nach dem Durchlaufen der Kammer wird der Schlauchrohling zur Erkaltung durch eine mit Wasser gefüllte Wanne, d.h. ein Wasserbad 11, geführt. Selbstverständlich sind auch andere Kühlungsmöglichkeiten für den infolge des Beschichtungsvorganges stark erhitzten Schlauch möglich, etwa mittels Glykol oder Luft.

Nach dem Heraustreten des beschichteten Schlauchrohlings aus dem Wasserbad 11 wird dieser über einen Abzug 15, welcher aus drei Walzen besteht, geführt. Hierbei sind die Walzen 15a und 15b fest gelagert, während die Walze 15c herauf und hinunter bewegt werden kann. Der Abzug 15 erzeugt neben einem Spannen des Schlauches durch Hinunterbewegen der Walze 15c auch eine Förderbewegung des Schlauches durch Drehung einer seiner Walzen, sodaß der Schlauchrohling 10 in Richtung 7, also entlang seiner Kettfäden, durch die Kammer 9 gezogen wird. Nach dem Durchlaufen des Abzugs wird der Schlauch durch eine Druckvorrichtung 27 mit einem Schriftzug versehen und auf einer Haspel bzw. Schlauchtrommel 13 aufgewickelt.

Das Innere der Kammer 9 wird durch eine Schmelzeleitung 14 mit dem Ausgang 18 eines Extruders 19 verbunden, welcher über einem Einfülltrichter bzw. Zuführmittel 20 für thermoplastischen Rohstoff, etwa in Form von Granulat, verfügt. Durch den Einfülltrichter und eine daran anschließende Leitung wird der thermoplastische Rohstoff 21 in eine Kompressionskammer 22 geleitet. Innerhalb der Kompressionskammer befindet sich eine von einem Motor 23 getriebene Schnecke 24. Die Schnecke komprimiert in für die Extrusionstechnik bekannter Weise den thermoplastischen Rohstoff zu Kunststoffschmelze und leitet die Schmelze durch den Ausgang 18 des Extruders zu Schmelzeleitungen 14, welche in das Innere der Kammer 9 führen. Sowohl der Extruder 19 als auch die Kammer 9 sind über ihren Umfang verteilt mit mehreren Heizeinrichtungen versehen, welche eine erhöhte Temperatur des Extruders, der Kammer sowie des darin befindlichen Kunststoffes erzeugen.

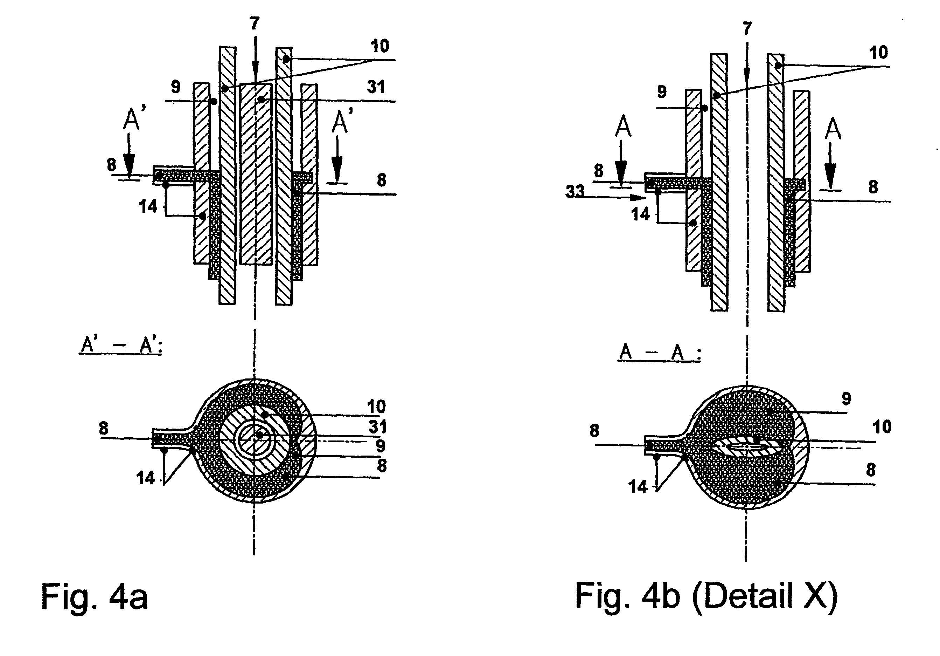

Fig. 4a zeigt eine Beschichtungskammer nach dem Stand der Technik. Hierin wird der Schlauchrohling 10 in Richtung 7 durch die Kammer 9 gezogen. Im Inneren des Schlauchrohlings 10 befindet sich dabei ein Dorn 31. Ein nicht dargestellter Extruder führt über z.B. herzkurvenförmige Schmelzeleitungen Kunststoffschmelze in das Innere der Kammer 9, sodaß die Außenfläche 10 des Schlauches mit Kunststoffschmelze 8 bedeckt wird. Bedingt durch den hohen Druck in der Kammer 9 wird der Schlauchrohling 10 von der Schlauchaußenseite her mit Druck belastet, sodaß die Innenfläche des Schlauchrohlings 10 einen starken Druck auf den Dorn 31 ausübt. Da der Schlauchrohling 10 mittels nicht dargestellter Vorrichtungen durch die Kammer 9 gezogen wird, kommt es im Bereich der Schnittstelle zwischen der Innenfläche des Schlauchrohlings und dem Dorn 31 zu einer starken Reibungskraft, welche der Kraft zum Durchführen des Schlauchrohlings in Richtung 7 durch die Kammer 9 entgegengesetzt ist. Bedingt durch diese entgegengesetzten Kräfte wird der im Bereich der Kammer befindliche Schlauchrohling stark in Längsrichtung gedehnt und in diesem gedehnten Zustand beschichtet. Bei einem Nachlassen der Längsspannung kann das Gewebe des Schlauchrohlings sich nicht mehr in den ungedehnten Ausgangszustand zurückbewegen, da das Gewebe durch die eingebrachte Beschichtung daran gehindert wird. Dies führt dazu, daß mit dem Verfahren nach dem Stand der Technik, selbst unter Einsatz hochelastischer Fasern in Längsrichtung des Schlauchrohlings, ein εₘₐₓ von max. 35 bis 40 % erreichbar wäre.

Fig. 4b zeigt eine erfindungsgemäße Kammer 9. Diese Fig. 4b zeigt eine erfindungsgemäße Kammer 9. Diese ist im Wesentlichen kreisförmig ausgeführt und verfügt über eine Schmelzeleitung 14, welche an einem nicht dargestellten Extruderausgang angeschlossen ist. Durch druckbehaftetes Einleiten von Kunststoffschmelze in Richtung 33 gelangt Kunststoffschmelze 8 in das Innere der Kammer 9. Oberhalb der Kammer 9 ist eine schlitzförmige Öffnung zum Einführen des flachen Schlauchrohlings vorgesehen. Am unteren Ausgang der Kammer 9 ist ein nicht dargestellter Abstreifer zur Vergleichmäßigung der Beschichtung auf dem beschichteten Schlauchrohling 10 vorgesehen. Der Schlauchrohling 10 wird durch nicht näher dargestellte Mittel in Richtung 7, d.h. in Längsrichtung und in Richtung seiner Kettfäden eines Gewebes 3, im Schlauchinneren ungestützt, durch die Kammer 9 geführt. Hierbei wird die Außenfläche des Schlauchrohlings mit durch die Schmelzeleitungen in das Kammerinnere eingeleitete Kunststoffschmelze 8 beschichtet. Die in Richtung 33 eingeführte Kunststoffschmelze 8 wird durch die Bewegung des Schlauchrohlings 10 in Führungsrichtung 7 mit in Führungsrichtung gezogen, sodaß im oberen Bereich der Kammer 9 keine weiteren Abdichtungsmaßnahmen notwendig sind.

Schnitt A-A in FIG 4b zeigt einen Querschnitt durch die Kammer 9. Hierin ist zu sehen, wie der Schlauchrohling 10 infolge der Einwirkung der in das Kammerinnere eintretende Kunststoffschmelze 8 flach zusammengepreßt wird bzw. in seinem im Wesentlichen flachen Zustand verbleibt. Im vorliegenden Ausführungsbeispiel ist die Kammer 9 im Querschnitt im Wesentlichen kreisförmig ausgeführt, es ist jedoch auch möglich, die Kammer 9 quer zur Längsrichtung des durchgeführten Schlauchrohlings 10 in einem zu dem Querschnitt des flachliegenden Schlauchrohlings korrespondierenden Querschnitt auszuführen.

Die Schmelzeleitungen 14, welche in das Kammerinnere führen, können im Endbereich, d.h. in dem zu dem Äußeren des Schlauchrohlings 10 hin orientierten Nahbereich, verschiedene Formen aufweisen. Es ist möglich, die Schmelzeleitungen entlang der Innenwand der Kammer als sich von dem Ausgang des Extruders 18 erstreckende Herzkurve auszuführen. Vorteilhaft ist es jedoch, die Kunststoffschmelze in Form eines Wendelverteilers an die Oberfläche des Schlauchrohlings heranzuführen. Hiermit wird stets gewährleistet, daß die Wege der Kunststoffschmelze von dem Ausgang des Extruders 18 bis zu jedem Punkt auf dem äußeren Umfang des Schlauchrohlings 10 etwa gleich lang sind. Hiermit wird die Bildung von Toträumen der Kunststoffschmelze 8 vermieden.

Da der Schlauchrohling 10 im Schlauchinneren ungestützt durch die Kammer geführt wird, entsteht keine Relativbewegung und daher keine Reibung im Schlauchrohlinginneren, welche zu einer Längsdehnung des Schlauchrohlings führen könnte. Somit wird der Schlauchrohling 10 im nahezu ungedehnten Zustand mit Kunststoffschmelze 8, welche zu einer Beschichtung 6 erkaltet, belegt. Es ist daher möglich, einen Schlauchrohling, welcher in Längsrichtung ausgerichtete hochelastische Kettfäden aufweist, so zu beschichten, daß auch nach dem Beschichtungsvorgang die Längsdehnbarkeit des Schlauchrohlings praktisch voll ausgenutzt werden kann. So kann z.B. bei einem Schlauchrohling, welcher in Längsrichtung ein εₘₐₓ im Bereich von 300 bis 350 % aufweist, ein εₘₐₓ des beschichteten Schlauches von 250 bis 300 %, etwa bei Verwendung eines thermoplastischen Polyurethan-Elastomeres als Beschichtungsmaterial problemlos erreicht werden. Diese hohen Elastizitäten können bei Beibehaltung einer sehr glatten und porenfreien Oberfläche erreicht werden, die Oberflächengüte der Beschichtung ist bei dem erfindungsgemäßen Verfahren gegenüber dem einleitend beschriebenen Tauch- oder Flachbeschichtungsverfahren deutlich verbessert.

## Patentansprüche

1. Schlauch (1) zur Auskleidung von Rohrleitungen und Kanälen, welcher in eine Rohrleitung (2) eingebracht wird, wobei der Schlauch (1) aus einem beschichteten Gewebe aus hochelastischen Kettfäden (4) sowie Schußfäden (5) geringerer Elastizität besteht.
**dadurch gekennzeichnet,**
**daß** das Verhältnis von εₘₐₓ der Kettfäden (4) zu εₘₐₓ der Schußfäden (5) im Bereich von 12 bis 40 liegt.

2. Schlauch nach Anspruch 1, worin die Kettfäden (4) aus Polyurethangarn sind.

3. Schlauch nach Anspruch 1, worin ein Kettfaden (4) aus einem mit einem, aus Polyester oder Polyamid bestehenden Windungsgarn umwundenen Grundkettfaden aus Polyurethan besteht.

4. Schlauch nach Anspruch 1, worin ein Kettfaden (4) aus einem mit einem Ümwindungsgarn umwundenen Grundkettfaden besteht, wobei
der Grundkettfaden eine längenbezogene Masse im Bereich von 22 bis 4000 dtex und
das Umwindungsgarn eine längenbezogene Masse von 44 bis 1000 dtex aufweist.

5. Schlauch nach Anspruch 1, worin der Kettfaden (4) aus einem mehrfach mit Umwindungsgarn umwundenen Grundkettfaden besteht.

6. Schlauch nach Anspruch 1, worin ein einzelner Kettfaden (4) ein εₘₐₓ im Bereich von 50 bis 500 % und ein Fₘₐₓ von 1 bis 100 N aufweist.

7. Schlauch nach Anspruch 1, worin der Schußfaden (5) aus Polyestergarn besteht.

8. Schlauch nach Anspruch 1, worin der Schußfaden (5) texturiert oder verwirbelt ist.

9. Schlauch nach Anspruch 1, worin der Schußfaden (5) eine längenbezogene Masse von 10 bis 1000 dtex, ein εₘₐₓ im Bereich von 1 bis 50 % und ein Fₘₐₓ im Bereich von 1 bis 100 N aufweist.

10. Schlauch nach Anspruch 1, worin ein Schußfaden (5) wechselweise unterhalb und oberhalb von längsgerichtet benachbarten Kettfäden (4) geführt ist.

11. Schlauch nach Anspruch 1, worin das Gewebe in der Ebene eine Dichte von 10 bis 1000 längsgerichtet benachbarten Kettfäden (4) pro dm und 10 bis 1000 längsgerichtet benachbarten Schußfäden (5) pro dm aufweist.

12. Schlauch nach Anspruch 1, worin εₘₐₓ des Gewebes bei Zug in Richtung der Kettfäden (4) im Bereich von 5 bis 500 % liegt.

13. Schlauch nach Anspruch 1, worin das Gewebe mit einem thermoplastischen Kunststoff (6) hoher Flexibilität beschichtet ist.

14. Schlauch nach Anspruch 1, worin der Schlauch (1) mit einem Polyurethan, insbesondere in seiner Ausführung als thermoplastisches Polyurethan-Elastomer (TPU), einem Polyester, insbesondere in seiner Ausführung als thermoplastisches Polyether-Ester-Elastomer (TPE), einem Polyolefin einschließlich Polyethylen (PE), einem Copolymer aus Ethylen und anderen α-Olefinen und thermoplastischen Polyolefin-Elastomeren (TPO), einem thermoplastischen Styrol-Butadien- bzw. Styrol-Ethylen-Propylen-Copolymer (TPS) oder einer Mischung aus Polypropylen und vernetzten Ethylen-Propylen-Dien-Copolymeren (TPV) beschichtet ist.

15. Schlauch nach Anspruch 1, worin εₘₐₓ der Beschichtung (6) im nicht auf einen Träger beschichteten Zustand im Bereich zwischen 80 und 800 % liegt.

16. Schlauch nach Anspruch 1, worin die Beschichtung (6) des Schlauches radial nur bis zu einem Teil der Dicke des Gewebes eingebracht ist.

17. Schlauch nach Anspruch 1, worin εₘₐₓ des Schlauches (1) in Längsrichtung im Bereich von 40 bis 400% liegt.

18. Schlauch nach Anspruch 1, worin das Verhältnis zwischen Berstdruck des Schlauches (1) und dem Reversionsdruck zum druckbehafteten Umstülpen des Schlauches (1) in eine Rohrleitung (2) im Bereich zwischen 1,1 und 4,4 liegt.

19. Schlauch nach Anspruch 1, worin der Außendurchmesser des Schlauches (1) im ungedehnten Zustand zwischen 9 und 1200 mm liegt.

20. Verfahren zur Herstellung eines Schlauches (1) nach Anspruch 1,
**dadurch gekennzeichnet, daß**
eine Kunststoffschmelze (8) in eine Kammer (9) gepreßt, und
ein Schlauchrohling (10) entlang seiner Längsachse und im Schlauchinneren ungestützt durch die Kammer (9) geführt wird,
wobei die Außenfläche des Schlauchrohlinges (10) mit der Kunststoffschmelze (8) beschichtet wird.

21. Verfahren nach Anspruch 20, worin der Schlauchrohling (10) aus Kett- (4) und Schußfäden (5) gewebt wird, wobei die nebeneinanderliegenden Kettfäden (4) längsgerichtet in Rohrform angeordnet werden und ein Schußfaden (5) bezüglich der Rohrform spiralförmig und in vorbestimmter Abfolge unter den Kettfäden (4) hindurch und über diese hinweg, unter Bildung eines Gewebeschlauches, geführt werden.

22. Verfahren nach Anspruch 20, worin der Schlauchrohling (10) aus einem Garn gewirkt ist.

23. Verfahren nach Anspruch 20, worin der Schlauchrohling (10) aus einem im wesentlichen rechteckförmigen ebenen Gewebe durch Zusammenfügen der Längsseiten des Rechtecks zu einer Rohrform gebildet ist.

24. Verfahren nach Anspruch 20, worin nach dem Durchlaufen der Kammer (9) die Beschichtungsstärke durch einen den Schlauch umgebenden Abstreifer vergleichmäßigt wird.

25. Verfahren nach Anspruch 20, worin der Schlauchrohling (10) nach dem Durchlaufen der Kammer (9) durch ein Wasserbad (11) geführt wird.

26. Verfahren nach Anspruch 20, worin der Schlauchrohling vor der Beschichtung von einer ersten Schlauchtrommel abgewickelt und nach der Beschichtung auf eine zweite Schlauchtrommel aufgewickelt wird.

27. Verfahren nach Anspruch 20, worin der Schlauch (1) nach dem Durchlaufen der Kammer (9) bedruckt wird.

28. Vorrichtung zur Herstellung eines Schlauches (1) nach Anspruch 1
**dadurch gekennzeichnet, daß**
eine Kammer (9) sowie
in das Kammerinnere führende Schmelzeleitungen (14) und
Mittel zum Durchführen eines Schlauchrohlings (10) in seiner Längsrichtung und im Schlauchinneren ungestützt durch die Kammer (9) vorgesehen sind,
wobei die Außenfläche des Schlauchrohlings (10) mit einer durch die Schmelzeleitungen (14) in das Kammerinnere eingeleiteten Kunststoffschmelze (8) beschichtet wird.

29. Vorrichtung nach Anspruch 28, worin die Kammer (9) quer zur Längsrichtung des durchgeführten Schlauchrohlings (10) einen zu dem Querschnitt des flachliegenden Schlauchrohlings (10) korrespondierenden Querschnitt aufweist.

30. Vorrichtung nach Anspruch 28, worin die Schmelzeleitungen (14) mit dem Ausgang eines Extruders (19) verbunden sind, welcher Zuführmittel (20) für thermoplastischen Rohstoff und eine damit verbundene Kompressionskammer (22) mit darin befindlicher motorgetriebener Schnecke (24) aufweist, wobei in der Kompressionskammer (22) der thermoplastische Rohstoff zu Kunststoffschmelze (8) komprimiert wird.

31. Vorrichtung nach Anspruch 28, worin die in das Kammerinnere führenden Schmelzeleitungen (14) in ihrem zu dem Schlauchrohling (10) hin orientierten Endbereich die Form einer Herzkurve aufweisen.

32. Vorrichtung nach Anspruch 28, worin die in das Kammerinnere führenden Schmelzeleitungen (14) in ihrem zu dem Schlauchrohling (10) hin orientierten Endbereich als Wendelverteiler ausgeführt sind.

33. Vorrichtung nach Anspruch 28, worin, bezogen auf die Durchlaufrichtung des Schlauchrohlings (10), hinter der Kammer (9) ein den beschichteten Schlauchrohling umfassender Abstreifer zum vergleichmaßigenden Abtrag überschüssiger Beschichtung (6) vorgesehen ist.

34. Vorrichtung nach Anspruch 28, worin zusätzlich bezogen auf die Durchlaufrichtung des Schlauchrohlings (10) ein hinter der Kammer (9) angeordnetes Wasserbad (11) zum Abkühlen des beschichteten Schlauchrohlings (10) und
Führungsmittel (17) zum Führen des Schlauches (1) durch das Wasserbad (11)
vorgesehen sind.

35. Vorrichtung nach Anspruch 28, worin zusätzlich eine, bezogen auf die Durchlaufrichtung des Schlauches (1), vor der Kammer (9) angeordnete erste Schlauchtrommel (12) zum Abwickeln des Schlauchrohlings (10) sowie eine nach der Kammer (9) angeordnete zweite Schlauchtrommel (13) zum Aufwickeln des beschichteten Schlauchrohlings (10) vorgesehen sind.

36. Vorrichtung nach Anspruch 28, worin, bezogen auf die Durchlaufrichtung des Schlauchrohlings (10), hinter der Kammer (9) eine Druckvorrichtung (27) zum Bedrucken des beschichteten Schlauchrohlings (10) angeordnet ist.

## Claims

1. A flexible tube (1) for lining pipes and ducts, which is introduced into a pipe (2), wherein the flexible tube (1) consists of a coated fabric of highly elastic warp threads (4) as well as weft threads (5) having a lower elasticity, **characterised in that** the ratio of εₘₐₓ of the warp threads (4) to εₘₐₓ of the weft threads (5) lies in the range of 12 to 40.

2. A flexible tube according to claim 1, wherein the warp threads (4) are of polyurethane yarn.

3. A flexible tube according to claim 1, wherein a warp thread (4) consists of a basic warp thread of polyurethane wrapped with a winding yarn consisting of polyester or polyamide.

4. A flexible tube according to claim 1, wherein a warp thread (4) consists of a basic warp thread wrapped with a covering yarn, wherein
the basic warp thread has a longitudinally related mass in the range of 22 to 4000 dtex and the covering yarn has a longitudinally related mass of 44 to 1000 dtex.

5. A flexible tube according to claim 1, wherein the warp thread (4) consists of a basic warp thread wrapped several times with covering yarn.

6. A flexible tube according to cairn 1, wherein an individual warp thread (4) has an εₘₐₓ in the region of 50 to 500 % and an Fₘₐₓ in the region to 1 to 100 N.

7. A flexible tube according to claim 1, wherein the weft thread (5) consists of polyester yarn.

8. A flexible tube according to claim 1, wherein the weft thread (5) is textured or interlaced.

9. A flexible tube according to claim 1, wherein the weft thread (5) has a longitudinally related mass of 10 to 1000 dtex, an εₘₐₓ in the range of 1 to 50 % and an Fₘₐₓ in the range of 1 to 100 N.

10. A flexible tube according to claim 1, wherein a weft thread (5) is alternately guided below and above longitudinally directed adjacent warp threads (4).

11. A flexible tube according to claim 1, wherein the fabric has a surface plane with a density of 10 to 1000 longitudinally directed adjacent warp threads (4) per dm and 10 to 1000 longitudinally directed adjacent weft threads (5) per dm.

12. A flexible tube according to claim 1, wherein εₘₐₓ of the fabric under tension towards the warp threads (4) lies in the range of 5 to 500 %.

13. A flexible tube according to claim 1, wherein the fabric is coated with a thermoplastic plastic (6) of a higher flexibility.

14. A flexible tube according to claim 1, wherein the flexible tube (1) is coated with a polyurethane, in particular with its design as a thermoplastic polyurethane elastomer (TPU), with a polyester, in particular with its design as a thermoplastic polyether-ester elastomer (TPE), with a polyolefine inlcuding polyethylene (PE), with a copolymer of ethylene and other α-olefines and thermoplastic polyolefine elastomers (TPO), a thermoplastic styrol-butadiene- or styrene-ethylene-propylene-copolymer (TPS) or a mxture of polypropylene and cross-linked ethylene-propylene-diene-copolymers (TPV).

15. A tubing according to claim 1, wherein εₘₐₓ in the condition of not being coated onto a carrier lies in the range between 80 and 800 %.

16. A flexible tube according to claim 1, wherein the coating (6) of the tubing is radially introduced only up to part of the thickness of the fabric.

17. A flexible tube according to claim 1, wherein εₘₐₓ of the flexible tube (1) longitudinally is within the range of 40 to 400%.

18. A flexible tube acording to claim 1, wherein the ratio between the bursting pressure of the flexible tube (1) and the reversing pressure for the pressurised turning inside out the flexible tube (1) into a pipe (2) lies in the range between 1.4 and 4.4.

19. A flexible tube according to claim 1, wherein the outer diameter of the flexible tube (1) in the unextended condition lies between 9 and 1200 mm.

20. A method for manufacturing a flexible tube (1) according to claim 1, **characterised in that** a plastic molten mass (8) is pressed into a chamber (9), and
a flexible tube blank (10) is guided along its longitudinal axis and not being supported in the interior of the flexible tube is guided through the chamber (9),
wherein the outer surface of the flexible tube blank (10) is coated with the plastic molten mass (8).

21. A method according to claim 20, wherein the flexible tube blank (10) is weaved from warp (4) and weft (5) threads, wherein the warp threads (4) lying next to one another are aligned longitudinally directed in the tube shape and a weft threads (5) with respect to the tube shape are led spirally and in a predetermined sequence under the warp threads (4) and over these whilst forming a woven flexible tube.

22. A method according to claim 20, wherein the flexible tube blank (10) is knitted from a yarn.

23. A method according to claim 20, wherein the flexible tube blank (10) is formed of an essentially rectangular planar woven fabric by joining together the longitudinal sides of the rectangle into a tube shape.

24. A method according to claim 20, wherein after running through the chamber (9) the coating density is made uniform by way of a scraper surrounding the flexible tube.

25. A method according to claim 20, wherein the flexible tube blank (10) after running through the chamber (9) is led through a water bath (11).

26. A method according to claim 20, wherein the flexible tube blank before the coating is wound of a first flexible tube drum and after the coating is wound onto a second flexible tube drum.

27. A method according to claim 20, wherein the flexible tube (1) after running through the chamber (9) is printed.

28. A device for manufacturing a flexible tube (1) according to claim 1, **characterised in that** there are provided
a chamber (9) as well as
melt conduits (14) leading into the inside of the chamber and
means for leading a flexible tube blank (10), in its longitudinal direction and not being supported in the interior of the flexible tube, through the chamber (9),
wherein the outer surface of the flexible tube blank (1) is coated with a plastic molten mass (8) introduced through the melt conduits (14) into the inside of the chamber.

29. A device according to claim 28, wherein the chamber (9) transverse to the longitudinal direction of the led-through flexible tube blank (10) has a cross section corresponding to the cross section of the flatly lying flexible tube blank (10).

30. A device according to claim 28, wherein the melt conduits (14) are connected to the output of an extruder (19) which comprises a supply means (20) for thermoplastic raw material and a compression chamber (22) connected thereto with a motor-driven worm (24) located therein, wherein in the compression chamber (22) the thermoplastic raw material is compressed into a plastic molten mass (8).

31. A device according to claim 28, wherein the melt conduits (14) leading into the inside of the chamber, in their end region directed towards the flexible tube blank (10) have the shape of the cardioid curve.

32. A device according to claim 28, wherein the melt conduits (14) leading into the inside of the chamber, in their end region orientated towards to flexible tube blank (10) are designed as a helical distributor.

33. A device according to claim 28, wherein with regard to the run-through direction of the flexible tube blank (10), behind the chamber (9) there is provided a scraper embracing the coated flexible tube blank, for the uniform removal of excess coating (6).

34. A device according to 28, wherein additionally with regard to the run-through direction of the flexible tube blank (10) there is arranged a water bath (11) behind the chamber (9) for cooling the coated flexible tube blank (10) and guide means (17) for guiding the flexible tube (1) through the water bath (11).

35. A device according to claim 28, wherein additionally with regard to the run-through direction of the flexible tube (1), in front of the chamber (9) there is provided a first flexible tube drum (12) for winding off the flexible tube blank (10) as well as a second flexible tube drum (13) arranged after the chamber (9) for winding up the coated flexible tube blank (10).

36. A device according to claim 28, wherein, with regard to the run-through direction of the flexible tube blank (10), behind the chamber (9) there is arranged a print device (27) for printing the coated flexible tube blank (10).

## Revendications

1. Flexible (1) pour habiller des conduites tubulaires et des canaux, qui est inséré dans une conduite tubulaire (2), le flexible (1) étant constitué de fils de chaîne (4) ainsi que de fils de trame (5), ayant une élasticité plus faible, **caractérisé en ce que** le rapport de la valeur εₘₐₓ des fils de chaîne (4) à la valeur εₘₐₓ des fils de trame (5) se situe dans la gamme comprise entre 12 et 40;

2. Flexible selon la revendication 1, dans lequel les fils de chaîne (4) sont formés d'un fil de polyuréthane.

3. Flexible selon la revendication 1, dans lequel un fil de chaîne (4) est constitué par un fil de chaîne de base en polyuréthane, autour duquel est enroulé un fil d'enroulement formé de polyester ou de polyamide.

4. Flexible selon la revendication 1, dans lequel un fil de chaîne (4) est constitué par un fil de chaîne de base autour duquel est enroulé un fil d'enroulement, et dans lequel le fil de chaîne de base possède une masse rapportée à la longueur, dans la gamme de 22 à 4000 dtex et le fil d'enroulement possède une masse, rapportée à la longueur, comprise entre 44 et 1000 dtex.

5. Flexible selon la revendication 1, dans lequel le fil de chaîne (4) est constitué par un fil de chaîne de base autour duquel un fil d'enroulement est enroulé de façon multiple.

6. Flexible selon la revendication 1, dans lequel un fil de chaîne individuel (4) possède une valeur εₘₐₓ dans la gamme comprise entre 50 et 500 % et une valeur Fₘₐₓ comprise entre 1 et 100 N.

7. Flexible selon la revendication 1, dans lequel le fil de trame (5) est formé d'un fil de polyester.

8. Flexible selon la revendication 1, dans lequel le fil de chaîne (5) est texturé ou est entremêlé.

9. Flexible selon la revendication 1, dans lequel le fil de chaîne (5) possède une masse, rapportée à la longueur, comprise entre 10 et 1000 dtex, une valeur εₘₐₓ située dans la gamme entre 1 et 50 % et une valeur Fₘₐₓ située dans la gamme située entre 1 et 100 N.

10. Flexible selon la revendication 1, dans lequel un fil de trame (5) est guidé alternativement au-dessus et au-dessous de fils de chaîne (4) voisins dans la direction longitudinale.

11. Flexible selon la revendication 1, dans lequel le tissu possède dans le plan une densité de 10 à 1000 filaments de chaîne (4) voisins dans la direction longitudinale par dm et 10 à 1000 filaments de trame (5) voisins dans la direction longitudinale par dm.

12. Flexible selon la revendication 1, dans lequel la valeur εₘₐₓ du tissu dans le cas d'une traction dans la direction des fils de chaîne (4) est située dans la gamme comprise entre 5 et 500 %.

13. Flexible selon la revendication 1, dans lequel le tissu est recouvert d'une matière thermoplastique (6) possédant une grande flexibilité.

14. Flexible selon la revendication 1, dans lequel le flexible (1) est recouvert par un polyuréthane, notamment dans sa forme de réalisation sous la forme d'une élastomère de polyuréthane thermoplastique (TPU), par un polyester, et notamment dans sa forme de réalisation sous la forme d'un élastomère de polyéther-ester (TPE), par une polyoléfine y compris le polyéthylène (PE), par un copolymère d'éthylène et d'autres α-oléfines et des élastomères de polyoléfines thermoplastique (TPO) par un copolymère thermoplastique styrène-butadiène ou styrène-éthylène-propylène (TPS) ou par un mélange de polypropylènes et de copolymères éthylène-propylène-diène (TPV) réticulés.

15. Flexible selon la revendication 1, dans lequel la valeur εₘₐₓ du revêtement (6) est dans l'état non appliqué sur un support, est située dans une gamme comprise entre 80 et 800 %.

16. Flexible selon la revendication 1, dans lequel le revêtement (6) du flexible est inséré radialement uniquement jusqu'à une partie de l'épaisseur du tissu.

17. Flexible selon la revendication 1, dans lequel la valeur εₘₐₓ du flexible (1) dans la direction longitudinale est comprise dans la gamme comprise entre 40 et 400 %.

18. Flexible selon la revendication 1, dans lequel le rapport entre la pression d'éclatement du flexible (1) et la pression d'inversion pour le retournement, affecté par la pression du flexible (1) dans une conduite tubulaire (2) est situé dans la gamme comprise entre 1,1 et 4,4.

19. Flexible selon la revendication 1, dans lequel le diamètre extérieur du flexible (1) à l'état non dilaté est compris entre 9 et 1200 mm.

20. Procédé pour fabriquer un flexible (1) selon la revendication 1, **caractérisé en ce**
**qu'**on comprime une masse de matière plastique (8) dans une chambre (9), et
**qu'**on guide une ébauche de flexible (10) le long de son axe longitudinal sans support à l'intérieur du flexible, à travers la chambre (9),
la surface extérieure de l'ébauche de flexible (10) étant recouverte par la masse fondue de matière plastique (8).

21. Procédé selon la revendication 20, selon lequel l'ébauche de flexible (10) est tissée avec des fils de chaîne (4) et des fils de trame (5), les fils de chaîne (4) disposés côte-à-côte étant orientés dans la direction longitudinale sous la forme d'un tube, et un fil de trame (5) s'étendant avec une forme hélicoïdale par rapport à la forme tubulaire et selon une séquence prédéterminée au-dessous des fils de chaîne (4) et au-dessus de ces derniers, en formant un flexible tissé.

22. Procédé selon la revendication 20, selon lequel l'ébauche de flexible (10) est formée par tricotage d'un fil.

23. Procédé selon la revendication 20, selon lequel l'ébauche de flexible (10) est formée par un tissu plat de forme essentiellement rectangulaire, par réunion des côtés longitudinaux du rectangle pour former une forme tubulaire.

24. Procédé selon la revendication 20, selon lequel après passage dans la chambre (9), l'épaisseur du revêtement est uniformisée au moyen d'une racle entourant le flexible.

25. Procédé selon la revendication 20, selon lequel l'ébauche de flexible après avoir traversé la chambre (9), on fait passer l'ébauche de flexible (10) dans un bain-marie (11).

26. Procédé selon la revendication 20, selon lequel on déroule l'ébauche de flexible d'un premier tambour à flexible avant d'un revêtement et, après le revêtement, on l'enroule sur un second tambour à flexible.

27. Procédé selon la revendication 20, selon lequel on comprime le flexible (1) après traversée de la chambre (9).

28. Dispositif pour fabriquer un flexible (1) selon la revendication 1, **caractérisé en ce qu'**il est prévu une chambre (9) ainsi que des canalisations (14) pour la masse fondue, qui aboutissent à l'intérieur de la chambre, et des moyens pour faire passer l'ébauche de flexible (10) dans sa direction longitudinale et d'une manière non supportée en son intérieur, à travers la chambre (9), la surface extérieure de l'ébauche de flexible (10) étant recouverte par une masse fondue de matière plastique (8) introduite à l'intérieur de la chambre par les canalisations (14) pour la masse fondue.

29. Dispositif selon la revendication 28, dans lequel la chambre (9) possède, transversalement par rapport à la direction longitudinale de l'ébauche de flexible (10) circulante, une section transversale qui correspond à la section transversale de l'ébauche de flexible (10) disposée à plat.

30. Dispositif selon la revendication 28, dans lequel les canalisations (14) pour la masse fondue sont reliées à la sortie d'une extrudeuse (19), qui comporte des moyens d'amenée (20) pour la matière plastique brute et une chambre de compression (22) reliée à ces moyens et comportant une vis (24) située dans cette chambre et entraînée par un moteur, la matière thermoplastique brute étant comprimée dans la chambre de compression (22) pour former la masse de matière plastique fondue (8).

31. Dispositif selon la revendication 28, dans lequel les canalisations (14) pour la masse fondue, qui aboutissent à l'intérieur de la chambre, possèdent, dans leur partie d'extrémité orientée en direction de l'ébauche de flexible (10) la forme d'une cardioïde.

32. Dispositif selon la revendication 28, dans lequel les canalisations (14) pour la masse fondue, qui aboutissent à l'intérieur de la chambre, sont réalisées, dans leur partie d'extrémité dirigée vers l'ébauche de flexible (10), sous la forme d'un répartiteur hélicoïdal.

33. Dispositif selon la revendication 28, dans lequel une racle, qui entoure l'ébauche de flexible pourvue d'un revêtement, en arrière de la chambre (9) dans la direction de circulation de l'ébauche de flexible (10), pour retirer, d'une manière réalisant une égalisation, le revêtement en excès (6).

34. Dispositif selon la revendication 28, dans lequel un bain-marie (11) disposé en arrière de la chambre (9), d'une manière rapportée à la direction de circulation de l'ébauche de flexible (10), pour refroidir l'ébauche de flexible (10) pourvue d'un revêtement, et des moyens de guidage (17) pour guider le flexible (1) à travers le bain-marie (11).

35. Dispositif selon la revendication 28, dans lequel il est en outre prévu un premier tambour à flexible (12), disposé en avant de la chambre (9) d'une manière rapportée à la direction de circulation du flexible (1), pour dérouler l'ébauche de flexible (10), ainsi qu'un second tambour à flexible (13) disposé en arrière de la chambre (9), pour enrouler l'ébauche de flexible (10) pourvue d'un revêtement.

36. Dispositif selon la revendication 28, dans lequel un dispositif d'impression (27) servant à appliquer une impression à l'ébauche de flexible (10) pourvue d'un revêtement est disposé en arrière de la chambre (9) dans la direction de circulation de l'ébauche de flexible (10).
